# EUROPEAN PATENT SPECIFICATION

(11) **EP 1 598 066 B1**
(45) Date of publication and mention of the grant of the patent: **16.12.2009**
(21) Application number: 03808657.5
(22) Date of filing: 30.07.2003
(51) Int. Cl.: A61K 31/404, A61K 9/14, A61P 25/06

(54) **POWDER INHALANT OF SUMATRIPTAN AND THE METHOD OF PREPARATION THEREOF**
PULVERINHALIERMITTEL MIT SUMATRIPTAN UND HERSTELLUNGSVERFAHREN DAFÜR
INHALANT EN POUDRE A BASE DE SUMATRIPTAN ET SON PROCEDE DE PREPARATION

(30) Priority: 16.10.2002 CN 02137472
(43) Date of publication of application: 23.11.2005
(73) Proprietor: Shanghai Institute of Pharmaceutical Industry, Shanghai 200040 (CN); Shanghai SIPI Pharmaceutical Co., Ltd, shanghai 200040 (CN)
(72) Inventor: JIN, Fang, Shanghai 200040 (CN); KONG, Xusheng, 6/F Wanji Bldg, No.4 Shuibeiyi Road, Shenzhen 518080 (CN)
(74) Representative: Humphreys, Ceris Anne
(86) International application number: PCT/CN2003/000611
(87) International publication number: WO 2004/035046

(56) References cited:
- EP-A- 0 496 307
- WO-A-00/06161
- WO-A-97/42992
- WO-A-98/35888
- WO-A-03/035034
- CN-A- 1 225 018
- US-A- 5 037 845

## Description

### Field of Invention

The invention relates to a nasal powder inhalant. In specific, it relates to the powder inhalant of sumatriptan and the method of preparation thereof.

### Technical Background

Powder Inhalation (PI) refers to a drug delivery system, from which one or more active ingredients, in the form of powder, are delivered into the respiratory system via special drug delivery devices and act locally or systemically. It is specifically targeted, rapid and highly effective, and has less side effects. According to different application methods, PI can be categorized into nasal powder inhalation and oral (pulmonary) powder inhalation. PI can be effectively used in the inhalation or spray of either low or high dose volume of drugs. As a drug form of which drugs are absorbed via abundant capillary vessels under the respiratory mucosa, PI is featured by the following characteristics. 1.No degradation in gastrointestinal tract; 2.No liver first-pass effect; 3.The medication is absorbed fast, and takes effect quickly; 4.The bioavailability of large molecule drugs can be improved by utilizing absorption accelerator or other means; 5.Small molecule drugs are especially suitable for direct delivery in respiratory tract via inhalation or spray; 6.After absorption, the drug can enter into systemic circulation directly to give systemic treatment; 7.The hydrophilic drug that can hardly be absorbed in gastrointestinal tract could be carried; 8.Good user-friendliness, especially for patients that have to take injections chronically; 9.Dose of locally acting drugs is significantly lowered, and therefore the side effects observed with the inhaled drug are far less than that with oral regimens.

With the prohibition of the use of Freon, what has attracted the attention of people in the area of pharmaceuticals around the world is searching for substitutes of Freon used in aerosols, as well as for new respiratory drug delivery forms. PI is a new pharmaceutical dosage form developed on the basis of aerosol together with the application of the knowledge in powder engineering technology and pharmaceutics so as to overcome the defects of aerosols. PI is easy to use and contains no propellant. The active ingredients are stable and face less disturbing factors as they are in solid state. These contribute to the increasing interests of the pharmacist to PI.

Up to now, all commercial PI products are for asthma, for example, salbutamaol or terbutaline given by inhaler for bronchodilation, and beclomethasone dipropionate given for chronic asthma. And all these are oral/pulmonary powder inhalation. Nasal powder inhalation is a new drug form of great growth potential. Main drugs and biological adherents are made into microsphere by biological adherence technologies. The micro-particles, delivered into nasal cavity via special device, absorb water, swell and adhere to nasal mucosa. Drugs are released in a longer period and bioavailability is improved. There are very few nasal PI in market. In mid-1980's, a nasal powder spray device (Puvlizer) was developed in UK and Japan for the nasal delivery of disodium cromoglycate and dihydroergotamine (for migraine). Probably due to the defects of the delivery device, those drugs were not widely applied. In mid-1990's, two famous manufacturers of drug delivery devices, Pfeiffer in Germany and Valois in France, developed unit-dose and bi-dose nasal powder delivery devices that are simple in design, reliable and easy to use. However, there has been no research report or patent application on drugs using these devices, nor has there been drugs launched with these devices.

Oral administration is the primary delivery route for most drugs. However, many drugs can be degraded, such as gastroenteric enzymatic degradation, metabolism, and liver first-pass effect, before entering into systemic circulation. With the progress of bio-technology, more and more peptide and protein drugs are used for treating diseases. In general, these drugs are not suitable via oral and have to be taken by injection, which is expensive, user-unfriendly and not suitable for long-term treatment. People in pharmaceutical area around the world therefore dedicate to the research of non-injection delivery forms for peptides. Nasal delivery came into attention in late 1980' s and early 1990's and became a hot research topic in early 1990's. About ten systemic-acting nasal spray drugs have been developed. All the drugs are in the form of solution and use mechanical spray pumps (unit-dose/bi-dose or multi-dose). However, as listed below, nasal spray drugs in the form of solution have some disadvantages.
1. Drugs in liquid state are not stable. Mechanical spray pumps may have physical or chemical interactions with some drugs. The spray pumps and the solution therefore have to be packaged individually and be assembled before application. Once opened, the drug has to be used up within certain time frame (2 weeks).
2. Multi-dose spray drugs have to be pre-sprayed before first application and will have some remains after the last application, which result in waste of expensive drugs. While unit-dose or bi-dose spray packages are too expensive.

The unique advantages of powder inhalation have drawn more and more research interests from pharmacy scientists. With the development of bio-technology and gene-technology, more and more peptide and protein are used clinically. Nasal and pulmonary drug delivery becomes an important non-injection delivery route for peptides, while powder inhalation is one of the drug forms of the greatest potential and competitiveness.

Because of the need for parallel development of the formulation and the device, the development of a PI formulation can be very complex. There are many factors that can influence the curative effects of PI, including: 1.The characteristics and composition of the powder formulation. Different delivery routes have different requirements on particle size. For instance, the main drug particle size should be less than 5µm for pulmonary PI, while for nasal PI the particle size should be 30-150µm. Moreover, the drug powder particles should be of some fluidity to ensure the dose accuracy in filling and inhalation, as well as to get an optimal spray pattern in application. 2.The choice of inhalant devices. The device should be selected according to the features of the active ingredient. Multi-dose device should be used for drugs for long-term application, while unit-dose device could be used for instable drugs. 3.The physiological and pathological condition of the patients. The age, gender, height, shape and mucosal incretion are closely related to curative effects. Patients should also be trained for the proper use of powder inhalation.

The chemical name of sumatriptan as a drug for migraine is 3-[2-(dimethylamino)ethyl]-N-methyl-indole-5-methanesulfonamide succinate, and the molecular formula is C₁₄H₂₁N₃O₂S. Currently in market there are sumatriptan injection, tablet and nasal spray, such as Imigran (injection and tablet) of GSK. Nasal spray satisfies the needs of patients who are unwilling or unable to take injection. It is user-friendly and easy to use. The drug takes effect 15 min after nasal application, second to injection (10 min), while it takes 30 min for oral tablet. However, current sumatriptan nasal spray is in the form of multi-dose solution. The drug is not stable in liquid state, nor is it convenient to carry. Expensive drugs are wasted due to prime sprays before the first application and drug remains after the last application. Because of above limitations, sumatriptan only accounts for 8.5% of Rx drugs for migraine worldwide, although it is regarded as the best standard treatment for migraine ever since it has been launched to market. EP0496307 discloses a dry powder comprising sumatriptan and starch, the dry powder purporting to be suitable for intranasal administration.

### Description of the Invention

The technical problem that the present invention is to solve is the disclosure of a powder inhalation of sumatriptan and its preparation method to overcome the disadvantage of existing technology, such as low drug stability, inconvenience in carrying and application, and waste of expensive drugs due to pre-sprays before the first application and drug remains after the last application.

The powder inhalant of the present invention comprises sumptriptan, colloidal adherent(s), water insoluble binder(s), diluent(s) and surfactant(s) in the following weight percentages:

| | | |
|---|---|---|
| 1. | sumatriptan | 5-90% |
| 2. | colloidal adherent(s) | 1-95% |
| 3. | water insoluble binder(s) | up to 90% |
| 4. | diluent(s) | up to 94% |
| 5. | surfactant(s) | up to 2% |

wherein the colloidal adherent(s) refers to pharmaceutically applicable polymer which can absorb water to form colloids, and includes one or more selected from the group consisting of sodium alginate, acacia gum, glutin, shell-lac, hydroxypropyl methyl cellulose, hydroxypropyl cellulose, carboxymethyl cellulose, starch, sodium starch glycolate, pregelatinized starch, and α,β,γ-cyclodextrin.
the water insoluble binder(s) refers to macromolecule which can absorb water but can't dissolve in water, and includes one or more selected from the group consisting of microcrystalline cellulose, ethylic cellulose, methyl cellulose, chitin and acrylic acid resin,
the diluent(s) includes one or more selected from the group consisting of starch, lactose, mannitol, sorbitol, pregelatinized starch, and α,β,γ cyclodextrin; and
the surfactant(s) includes one or more selected from the group consisting of polysorbate, poloxamer, Myrj and Brij.

The surfactant is effective in reducing the absorption of active ingredient by the surface of the container and increasing the absorption of the drug by the patient.

The particle size is in the range from 10 to 200µm, and preferably from 30 to 100µm. At least 80% of the drug particles have the particle size from 30 to 100µm.

According to the present invention, carriers with good fluidity, such as lactose, mannitol, starch and cyclodextin can be added into the composition to ease filling and application.

The powder inhalation of sumatriptan of the present invention is indicated for the treatment of intermittent acute migraine. Being inhaled into the nose, the drug is absorbed by the nasal mucosa and acts systemically. The stability of the drug in form of solid powder and packaged in single dose is markedly improved compared with that in liquid form. The powder form is also easy to carry. In application, the powder particles can be entirely inhaled into nasal cavity, with little remains in the device, and can easily deposit at the absorption area. Migraine is an intermittent acute headache that normally happens 4-5 times a month. This preparation is especially suitable for patients with such disease. There are abundant capillary vessels in nasal mucosa, which contribute to the rapid effects after drug delivery and significant decrease of treatment expenditure for patients. Drug dosage is determined according to the ages and pathological conditions of the patients and is usually 5-10mg.

For convenient application, the powder inhalant of sumatriptan can be filled into powder delivery devices, such as the updated nasal bi-dose powder device developed by Pfeiffer GmbH. Packaged in a bi-dose blister, the drug can be easily kept in storage. The device can be re-used, and the blister of drug powder can be purchased upon demand. In using the device, patients actively inhale the drug powder particles. The patient presses the actuator to break the aluminum foil covering the blister, put the actuator close to the nose and inhale. Drug powder particles in the blister come into the nasal cavity and adhere to the nasal mucosa. The drug is absorbed via the nasal mucosa and takes effect. The device is simple in structure, small and easy to carry.

The preparation of the invention includes following steps.
1. Pulverize and mesh the main drug sumatriptan and adjuvant for later use.
2. Make the active ingredient and biological adherent materials, etc., into aggregation by spray drying or freeze drying technology, or normal granulating technology. Fill it into blisters in metered dosage, or uniformly mix it with carriers of good fluidity and fill into the blisters.

### Brief Description of the Drawings

- Figure 1: The curve of time-blood concentration of Sumatriptan
- Figure 2: Structure of bi-dose blister

### Embodiments

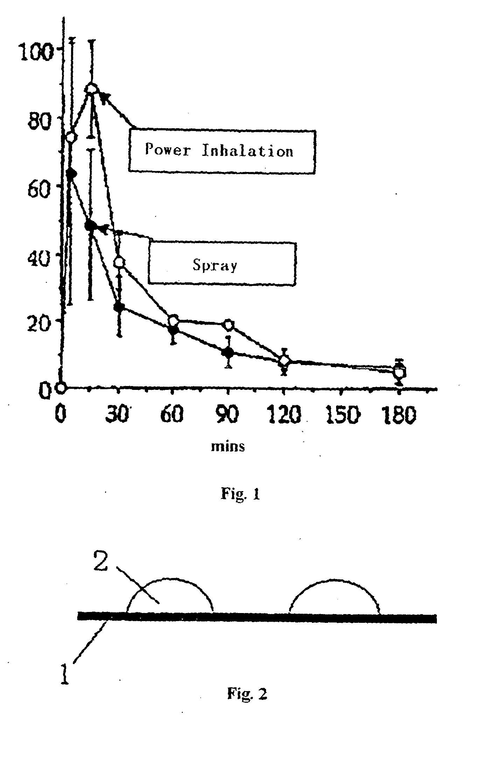

The drug of the invention can be filled into the bi-dose blisters as demonstrated in Figure 2 for easier application. The bi-dose blister includes motherboard 1 and two blisters to be filled with drugs on mother board 1.

### A. Irritative Experiments

1) Rabbits treated intra-nasally with Sumatriptan succinate powder, 42mg /rabbit dosage (equivalent to 30mg Sumatriptan) (equivalent to 52.5-210 times of one dosage/person by weight; 18-71 times of one dosage/person by body surface area), were used to carry out the experiments of the max dosage for nasal delivery. No rabbit died. So the max dosage for Sumatriptan succinate powder of intranasal administration is 42mg/rabbit (equivalent to 30mg Sumatriptan).
2) Rabbits treated intra-nasally with Sumatriptan succinate powder, 28mg /rabbit dosage, (equivalent to 20mg Sumatriptan) (equivalent to 48 times of one dosage/person by body surface area, 140 times of one dosage/person by weight), including single dosage of 20mg/rabbit and multiple dosage (continuously 7 days, total 140mg/rabbit). Preparation was sprayed into nasal cavities to carry out the experiments of irritation to the nasal mucosa. Morphological and pathology histological researches showed no obvious reaction or symptom which were relative to drug.

### B. Absorption Experiments for Animal

### Animals and methods:

12 rabbits were divided into 2 groups, one for nasal powder inhaler and the other for nasal liquid spray (6 in each group, 3 males and 3 females). Experiments were carried out after feeding the rabbits for 3 days. Fasting was required and the rabbits were fed only with water before experiments.
Dosage: 5mg/day.

### Methods of determination of sumatriptan in serum:

Concentration of sumatriptan in plasma and serum was determined by HPLC. Chromatographic instrumentation and conditions were as below:
HPLC column: C₁₈ reversed-phase HPLC column (125×4.6mm, 5µm);
Detector: electrochemical detector (Coulometric detector, +0.8v);
Mobile phase: phosphate buffer (5.25g Sodium phosphate dehydrate + 2.79g Potassium di-hydrogen phosphate + 1000ml distilled water, pH7.0)-methanol (40: 60, v/v);
Flow rate: 1ml/min.

### Method of extraction:

Add 1ml plasma into 100µl 4M sodium hydroxide. Extract with 2.6ml mixture of chloromethane and ethyl acetate (1: 4, v/v). Vortex mix for 15 min and centrifugate for 4 min. Precisely transfer 2ml top-layer into test tube. Add 2ml n-hexane and 300µl pH7.0 phosphate buffer. Vortex mix and centrifugate as above. Insert the test tube into the mixture of frozen acetone and solid carbon dioxide to freeze the under-layer which was the layer of aquatic phase. On the condition of low pressure, top-layer was transferred. When the liquid of under-layer was thawed, 200µl extract was transferred exactly and injected on to the HPLC for calculation.

### The time point of sampling:

Get 1ml blood sample at before administration (0min) and 5,15,30,60,120,180min respectively after administration. Plasma was separated and stored at -40°C. The results of determination were shown in Fig 1.

### Example 1

Crush 5g sumatriptan, 2g sodium carboxymethylcellulose, 3g cellulose microcrystallisate, 10g diluents (lactose) and 0.1g surfactant (poloxamer) respectively, and pass the 200 mesh screen. Dissolve it and then dry it by spray at 80°C. Select the agglomerates of standard particle diameter and put them into the blister as shown in figure 2. Thus the preparation of the invention is obtained.

### Example 2

Crush 5g sumatriptan and 2g colloidal adherent (sodium alginate), 2g colloidal adherent (hydroxypropyl cellulose), 2g water insoluble binder (chitin) and 10g diluents (5g β-cyclodextrin,10g mannitol) respectively, and pass the 200 mesh screen, take 1% polysorbate 80 water solution to make the adhesive granules and dry it at 60°C. Select the agglomerates of standard particle diameter and add 5g carrier (angle of repose of lactose<40°), then put them into the blister as shown in figure 2. Thus the preparation of the invention is obtained.

### Example 3 (not according to the invention)

Crush 5g sumatriptan and 2g colloidal adherent (hydroxypropyl methyl cellulose), 1g colloidal adherent (starch), 2g water insoluble binder (methyl cellulose) and 5g diluents (lactose) respectively, and pass the 200 mesh screen, take 75% ethanol-water (v/v) solution to make the adhesive granules and dry them at 60°C. Select the agglomerates of standard particle diameter and add 10g carrier (angle of repose of pregelatinized starch<40°), then put them into the blister as shown in figure 2.

### Example 4

Dissolve or suspend 5g sumatriptan and 1g colloidal adherent (acacia gum), 2g water insoluble binder (methyl cellulose), 5g diluents (lactose) and 0.2g Brij in water, freeze dry the solution then pass the 200 mesh screen. Select the agglomerates of standard particle diameter and add 15g carrier (angle of repose of lactose<40°), then put them into the blister as shown in figure 2. Thus the preparation of the invention is obtained.

## Claims

1. A dry powder inhalation of sumatriptan, the inhalation comprising sumatriptan, colloidal adherent(s), water insoluble binder(s), diluent(s) and surfactant(s) in the following weight percentage:
| | |
|---|---|
| sumatriptan | 5-90% |
| colloidal adherent(s) | 1-95% |
| water insoluble binder(s) | up to 90% |
| diluent(s) | up to 94% |
| surfactant(s) | up to 2% |
with the proviso that the amount must not exceed 100% and wherein, the said colloidal adherent(s) refers to pharmaceutically applicable polymer which can absorb water to form colloids and includes one or more selected from the group consisting of sodium alginate, acacia gum, glutin, shell-lac, hydroxypropyl methyl cellulose, hydroxypropyl cellulose, carboxymethyl cellulose, starch, sodium starch glycolate, pregelatinized starch, and α,β,γ-cyclodextrin;
the said water insoluble binder(s) refers to macromolecule which can absorb water but can't dissolve in water, and includes one or more selected from the group consisting of microcrystalline cellulose, ethylic cellulose, methyl cellulose, chitin and acrylic acid resin;
the said diluent(s) includes one or more selected from the group consisting of starch, lactose, mannitol, sorbitol pregelatinized starch, and α,β,γ-cyclodextrin; and
the said surfactant(s) includes one or more selected from the group consisting of polysorbate, poloxamer, Myrj and Brij.

2. A dry powder inhalation according to claim 1 wherein the inhalation is in the form of particles and particle size is from 10 to 200µm.

3. A dry powder inhalation according to claim 2 wherein the particle size is from 30 to 100µm.

4. A dry powder inhalation according to any one preceding claim wherein a fine fluid carrier is added to the inhalation and the said carrier comprises one selected from lactose, mannitol, starch and cyclodextrin.

5. A bi-dose blister filled with the dry powder inhalation according to any one of claims 1 to 4.

6. A method of preparation of the inhalation according to any one of claims 1 to 4, including the steps of:
(i) pulverise and mesh the sumatriptan, colloidal adherent, surfactant, diluent and water-insoluble binder;
(ii) aggregate the sumatriptan, colloidal adherent, surfactant, diluent and water-insoluble binder by spray drying or freezing drying technology, or normal granulating technology; and
(iii) select the agglomerates of standard particle size and fill them into blisters, or uniformly mix them with carriers of good fluidity and fill them into blisters.

## Patentansprüche

1. Inhalationsmittel für Sumatriptan in Form eines trockenen Pulvers, wobei das Inhalationsmittel Sumatriptan, ein oder mehrere kolloidale Haftmittel, ein oder mehrere wasserunlösliche Bindemittel, ein oder mehrere Verdünnungsmittel, und ein oder mehrere grenzflächenaktive Mittel in folgenden Gewichtsprozenten umfasst:
| | |
|---|---|
| Sumatriptan | 5 bis 90 Gew.-% |
| ein oder mehrere kolloidale Haftmittel | 1 bis 95 Gew.-% |
| ein oder mehrere wasserunlösliche Bindemittel | bis zu 90 Gew.-% |
| ein oder mehrere Verdünnungsmittel | bis zu 94 Gew.-% |
| ein oder mehrere grenzflächenaktive Mittel | bis zu 2 Gew.-%, |
mit der Maßgabe, dass die Menge 100 Gew.-% nicht überschreiten darf, und
wobei sich das oder die kolloidalen Haftmittel auf ein pharmazeutisch verträgliches Polymer beziehen, das Wasser absorbieren kann, um Kolloide zu bilden, und einen oder mehrere Stoffe umfasst, welche aus der Gruppe ausgewählt werden, die aus Natriumalginat, Akaziengummi, Glutin, Schellack, Hydroxypropyl-MethylZellulose, Hydroxypropyl-Zellulose, Carboxymethyl-Zellulose, Stärke, Natrium-Stärke-Glycolat, modifizierter Stärke und α-, β-, und γ-Cyclodextrin besteht;
sich das oder die wasserunlöslichen Bindemittel auf ein Makromolekül beziehen, das Wasser absorbieren kann, aber sich nicht in Wasser lösen kann, und einen oder mehrere Stoffe umfasst, welche aus der Gruppe ausgewählt werden, die aus mikrokristalliner Zellulose, Ethylzellulose, Methylzellulose, Chitin und Acrylsäureharzen besteht;
das oder die Verdünnungsmittel einen oder mehrere Stoffe umfassen, welche aus der Gruppe ausgewählt werden, die aus Stärke, Laktose, Mannit, Sorbit, modifizierter Stärke, und α-, β-, und γ-Cyclodextrin besteht;
und das oder die grenzflächenaktiven Mittel einen oder mehrere Stoffe umfassen, welche aus der Gruppe ausgewählt werden, die aus Polysorbat, Poloxamer, Myrj und Brij besteht.

2. Inhalationsmittel in Form eines trockenen Pulvers nach Anspruch 1, wobei das Inhalationsmittel in Form von Teilchen vorliegt und die Teilchengröße 10 bis 200 µm beträgt.

3. Inhalationsmittel in Form eines trockenen Pulvers nach Anspruch 2, wobei die Teilchengröße 30 bis 100 µm beträgt.

4. Inhalationsmittel in Form eines trockenen Pulvers nach einem der vorstehenden Ansprüche, wobei ein feiner flüssiger Träger zu dem Inhalationsmittel gegeben wird und der Träger einen Stoff umfasst, welcher aus Laktose, Mannit, Stärke und Cyclodextrin ausgewählt wird.

5. Blister mit zwei Dosen, gefüllt mit Inhalationsmittel in Form eines trockenen Pulvers nach einem der Ansprüche 1 bis 4.

6. Verfahren zur Herstellung des Inhalationsmittels nach einem der Ansprüche 1 bis 4, welches Verfahren die folgenden Schritte umfasst:
(i) Pulverisieren und Vermengen des Sumatriptans, des kolloidalen Haftmittels, des grenzflächenaktiven Mittels, des Verdünnungsmittels, und des wasserunlöslichen Bindemittels;
(ii) Aggregieren des Sumatriptans, des kolloidalen Haftmittels, des grenzflächenaktiven Mittels, des Verdünnungsmittels, und des wasserunlöslichen Bindemittels durch ein Sprühtrocknungs- oder Gefriertrocknungs-Verfahren oder durch ein normales Granulierverfahren; und
(iii) Auswählen der Agglomerate mit standardmäßiger Teilchengröße und Füllen derselben in Blister, oder gleichmäßiges Mischen derselben mit Trägern mit gutem Fließvermögen und Füllen derselben in Packungen.

## Revendications

1. Inhalation en poudre sèche au sumatriptan, l'inhalation comprenant du sumatriptan, un(des) adhésif(s) colloïdal(colloïdaux), un(des) liant(s) insoluble(s) dans l'eau, un(des) diluant(s) et un(des) tensioactif(s) selon le pourcentage pondéral suivant :
| | |
|---|---|
| sumatriptan | 5-90 % |
| adhésif(s) colloïdal(colloïdaux) | 1-95% |
| liant(s) insoluble(s) dans l'eau | jusqu'à 90 % |
| diluant(s) | jusqu'à 94 % |
| tensioactif(s) | jusqu'à 2 % |
à condition que la quantité totale n'excède pas 100 %, et dans laquelle le(s)dit(s) adhésif(s) colloïdal(colloïdaux) fait(font) référence à un polymère pharmaceutiquement applicable qui peut absorber de l'eau pour former des colloïdes et comprend(comprennent) l'un ou plusieurs membres choisi(s) dans le groupe constitué par l'alginate de sodium, la gomme arabique, la gélatine, la gomme laque, l'hydroxypropylméthylcellulose, l'hydroxy-propylcellulose, la carboxyméthylcellulose, l'amidon, le glycolate d'amidon sodique, l'amidon prégélatinisé, et les alpha-, beta- et gamma-cyclodextrines ;
le(s)dit(s) liant(s) insoluble(s) dans l'eau fait(font) référence à une macromolécule qui peut absorber de l'eau mais ne peut pas se dissoudre dans l'eau, et comprend(comprennent) l'un ou plusieurs membres choisi(s) dans le groupe constitué par la cellulose microcristalline, l'éthylcellulose, la méthylcellulose, la chitine et la résine acrylique ;
le(s)dit(s) diluant(s) comprend(comprennent) l'un ou plusieurs membres choisi(s) dans le groupe constitué par l'amidon, le lactose, le mannitol, l'amidon prégélatinisé de sorbitol, et les alpha-, beta- et gamma-cyclodextrines ; et
le(s)dit(s) tensioactif(s) comprend(comprennent) l'un ou plusieurs membres choisi(s) dans le groupe constitué par les polysorbates, les poloxamères, les Myrj et les Brij.

2. Inhalation en poudre sèche selon la revendication 1, dans laquelle l'inhalation se présente sous forme de particules, et la taille des particules est comprise entre 10 et 200 µm.

3. Inhalation en poudre sèche selon la revendication 2, dans laquelle la taille des particules est comprise entre 30 et 100 µm.

4. Inhalation en poudre sèche selon l'une quelconque des revendications précédentes, dans laquelle un véhicule fluide fin est ajouté à l'inhalation et ledit véhicule comprend l'un d'entre lactose, mannitol, amidon et cyclodextrine.

5. Emballage-coque bi-dose rempli avec l'inhalation en poudre sèche selon l'une quelconque des revendications 1 à 4.

6. Procédé de préparation de l'inhalation selon l'une quelconque des revendications 1 à 4, comprenant les étapes consistant à
(i) pulvériser et tamiser le sumatriptan, l'adhésif colloïdal, le tensioactif, le diluant et le liant insoluble dans l'eau ;
(ii) faire s'agglomérer le sumatriptan, l'adhésif colloïdal, le tensioactif, le diluant et le liant insoluble dans l'eau par une technique de séchage par atomisation ou par lyophilisation, ou par une technique de granulation normale ; et
(iii) choisir les agglomérats de taille de particule standard et en remplir des emballages-coques, ou bien les mélanger uniformément avec des véhicules de bonne fluidité et en remplir des emballages-coques.
